# EUROPEAN PATENT APPLICATION

(11) **EP 4 113 530 A1**
(43) Date of publication of application: **04.01.2023**
(21) Application number: 21182330.7
(22) Date of filing: 29.06.2021
(51) Int. Cl.: G16H 40/40

(54) **METHODS AND APPARATUSES FOR FACILITATING MAINTENANCE OF A MEDICAL ANALYZER DEVICE**

(71) Applicant: Radiometer Medical ApS, 2700 Brønshøj (DK)
(72) Inventor: DANIELSEN, Martin Tandal Srichan, 2700 Brønshøj (DK)
(74) Representative: Radiometer IPR

(57) **Abstract**

A computer-implemented method for facilitating maintenance of a medical analyzer device is disclosed. The method comprises obtaining a plurality of measurement results associated with a plurality of quality control (QC) measurements performed by a plurality of medical analyzer devices on a plurality of predefined QC samples. Each QC sample is associated with a predefined target range and each medical analyzer device is associated with a peer group of medical analyzer devices. The method further comprises, for each medical analyzer device, evaluating the obtained measurement results against measurement results of the associated peer group in order to identify a deviation of at least one QC measurement parameter. Then, if a deviation above a predefined threshold is identified, the method further comprises obtaining an action log of the medical analyzer device. The action log comprises data indicative of any previously transmitted maintenance actions associated with the medical analyzer device. Moreover, the method comprises obtaining at least one suggested maintenance action for the medical analyzer device associated with the identified deviation based on a type of QC measurement parameter that is associated with the identified deviation. The method further comprises, selecting a maintenance action for the medical analyzer device associated with the identified deviation from the at least one suggested maintenance action based on the obtained action log. Furthermore, the method comprises transmitting, to a device for managing the medical analyzer device associated with the identified deviation, data indicative of an instruction to execute the selected maintenance action for the medical analyzer device.

## Description

### TECHNICAL FIELD

The present disclosure relates generally to the field of clinical analysis and to devices configured to analyze biological samples. In particular, the present disclosure relates to quality control (QC) processes for such devices.

### BACKGROUND

Within the field of clinical analysis, a wide variety of electronic medical analyzers are known that allow clinical personnel to acquire test results and/or measurement results or otherwise analyze specimens such as samples of bodily fluids. These analyses include in vitro measurements on individual samples of e.g. whole blood, serum, plasma and urine, tissue samples or other types of samples obtained from a patient. Further, the analysis includes in vivo measurements on sample streams such as transcutaneous measurements of e.g. the partial pressures of oxygen (*p*O₂) and/or carbon dioxide (*p*CO₂) and also pulse oximetry measurements. Generally, a medical analyzer is a device configured to conduct chemical, optical, physical or similar analysis on specimen e.g. on individual samples or sample streams. Such medical analyzers include analyzers for performing various forms of clinical tests and/or analysis, such as the measurement of physiological parameters of a patient.

In modern clinical environments, medical analyzers are widely used and there is a trend of moving more and more tests from a central laboratory to the actual point of care (POC). Even though this has a number of advantages, it also involves a number of challenges. For example, the operational environment in which POC medical analyzers are operated is less controllable than the environment of a central laboratory, e.g. in terms of controlling the personnel operating the devices.

Further, modern medical analyzers require regular quality control (QC), which may be understood as a process used to monitor and evaluate the analytical process that produces the patient results. There are a number of techniques to test the functionality of medical analyzers for QC purposes. However, a common practice is to perform periodic QC tests where measurements are performed on a set of known standard samples - may also be referred to as control substance, QC specimen or QC samples - each having a predetermined characteristic, in order to verify that the measurement result falls within a predefined range of acceptable values for each QC sample. These predefined ranges are often referred to as a control ranges.

Commonly, there is one or more dedicated personnel - often referred to as Point of Care Coordinators (POCC) - tasked with analyzing the QC results in order to identify potential problems or future maintenance needs. In more detail, oftentimes the POCC is tasked with manually analyzing the measurement results obtained from the QC program, identifying immediate or upcoming problems, and then trying to find a suitable solution by studying a manual or set of instructions associated the particular analyzer. Moreover, in some instances, for a given problem (e.g. measurements related to a specific parameter drifting over time) a first POCC may attempt to resolve the problem by replacement of a suspected faulty hardware component in accordance with the associated instruction manual. Then, at a later moment in time when the same problem is identified by a second POCC (that isn't aware of the maintenance history of the device), the same hardware component is unnecessarily replaced again. Thus, not only are the state of the art maintenance procedures prone to drawbacks in terms of time efficiency, but they may also result in unnecessary discarding of components, which increases waste and associated costs of the devices.

There is therefore a need in the art for new solutions for facilitating maintenance of medical analyzers, which solve, or at least mitigate at least some of the above discussed problems.

### SUMMARY

It should be emphasized that the term "comprises/comprising" (replaceable by "includes/including") when used in this specification is taken to specify the presence of stated features, integers, steps, or components, but does not preclude the presence or addition of one or more other features, integers, steps, components, or groups thereof. As used herein, the singular forms "a", "an" and "the" are intended to include the plural forms as well, unless the context clearly indicates otherwise.

Generally, when an apparatus is referred to herein, it is to be understood as a physical product; e.g., a device. The physical product may comprise one or more parts, such as control circuitry in the form of one or more controllers, one or more processors, or the like.

It is an object of some embodiments to solve or mitigate, alleviate, or eliminate at least some of the above or other disadvantages.

A first aspect is a computer-implemented method for facilitating maintenance of a medical analyzer device. The method comprises obtaining a plurality of measurement results associated with a plurality of quality control (QC) measurements performed by a plurality of medical analyzer devices on a plurality of predefined QC samples. Each QC sample is associated with a predefined target range and each medical analyzer device is associated with a peer group of medical analyzer devices. The method further comprises, for each medical analyzer device, evaluating the obtained measurement results against measurement results of the associated peer group in order to identify a deviation of at least one QC measurement parameter.

Then, if a deviation above a predefined threshold is identified, the method further comprises obtaining an action log of the medical analyzer device. The action log comprises data indicative of any previously transmitted maintenance actions associated with the medical analyzer device. Moreover, the method comprises obtaining at least one suggested maintenance action for the medical analyzer device associated with the identified deviation based on a type of QC measurement parameter that is associated with the identified deviation. The method further comprises, selecting a maintenance action for the medical analyzer device associated with the identified deviation from the at least one suggested maintenance action based on the obtained action log. Furthermore, the method comprises transmitting, to a device for managing the medical analyzer device associated with the identified deviation, data indicative of an instruction to execute the selected maintenance action for the medical analyzer device.

In some embodiments, the step of obtaining at least one maintenance action for the medical analyzer device associated with the identified deviation comprises identifying a parameter group of the type of QC measurement parameter that is associated with the identified deviation, and obtaining at least one suggested maintenance action based on the identified parameter group.

Further, in some embodiments, each QC measurement parameter is associated with a parameter group out of a set of predefined parameter groups. The set of predefined parameter groups are formed based on one or more hardware and/or software components of the medical analyzer devices being associated with the performance of the QC measurement.

Still further, in some embodiments, the at least one suggested maintenance action comprises a plurality of suggested maintenance actions arranged in a sequential order, and the selected maintenance action is a first maintenance action in accordance with the sequential order. Accordingly, the method further comprises, if the deviation above the predefined threshold is identified, checking the obtained action log in order to identify an entry in the action log corresponding to a transmission of the first maintenance action. Then, if the entry in the action log corresponding to the transmission of the first maintenance action is identified and if the deviation above the predefined threshold is identified at a point in time after the entry in the action log corresponding to the transmission of the first maintenance action, the method further comprises transmitting, to the device for managing the medical analyzer device associated with the identified deviation, data indicative of an instruction to execute a second maintenance action of the plurality of suggested maintenance actions for the medical analyzer device. The second action is a subsequent maintenance action relative to the first maintenance action in accordance with the sequential order.

In some embodiments, the method further comprises obtaining a maintenance log of the medical analyzer device. The maintenance log comprises data indicative of historical maintenance actions performed on the medical analyzer device prior to a point in time defined by a timing of the obtained measurement results of the medical analyzer device. Moreover, after transmitting data indicative of the selected maintenance action for the medical analyzer device, the method further comprises checking the obtained maintenance log in order to identify an entry in the maintenance log corresponding to an execution of the selected maintenance action. Then, if the entry in the maintenance log corresponding to the execution of the selected maintenance action is not identified within a predefined time period, the method further comprises transmitting, to the device for managing the medical analyzer device associated with the identified deviation, data indicative of a reminder of the selected maintenance action for the medical analyzer device.

In some embodiments, the measurement results of the peer group comprises information about an average measurement value for each QC measurement associated with the peer group. Accordingly, in some embodiments, the method further comprises updating the average measurement value for each QC measurement of the peer group based on at least a portion of the obtained plurality of measurements.

In some embodiments, the predefined threshold comprises a first predefined threshold, and the step of evaluating the obtained measurement results comprises comparing the obtained measurement results with the average measurement value for each QC measurement of the associated peer group, and determining that a deviation is identified if the comparison indicates a difference above the first predefined threshold.

Moreover, in some embodiments, the obtained measurement results comprise information about an evolution over time of the measurement results for each of the plurality of medical analyzer devices and wherein the predefined threshold comprises a second predefined threshold. Accordingly, in some embodiments, the step of evaluating the obtained measurement results comprises comparing the measurement results for each QC measurement parameter for a most recent time period with an evolution over time of the average measurement value for each corresponding QC measurement of the associated peer group during the most recent time period and determining that a deviation is identified if the comparison indicates a difference above the second predefined threshold value during the most recent time period.

Further, in some embodiments, the obtained measurement results comprises information about an evolution over time of the measurement results for the plurality of medical analyzer devices. Accordingly, in some embodiments, the step of evaluating the obtained measurement results comprises comparing the measurement results for each QC measurement parameter for a most recent time period with an evolution over time of the average measurement value for each corresponding QC measurement during the most recent time period, and determining that a deviation is identified if the comparison indicates an increasing difference over time during the most recent time period.

A second aspect is a (non-transitory) computer-readable storage medium storing one or more programs configured to be executed by one or more processors of a processing system, the one or more programs comprising instructions for performing the method of the first aspect according to any one of the embodiments disclosed herein. With this aspect, similar advantages and preferred features are present as in the previously discussed first aspect.

The term "non-transitory," as used herein, is intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link. Thus, the term "non-transitory", as used herein, is a limitation of the medium itself (i.e., tangible, not a signal) as opposed to a limitation on data storage persistency (e.g., RAM vs. ROM).

A third aspect is an apparatus for facilitating maintenance of a medical analyzer device. The apparatus comprises control circuitry configured to obtain a plurality of measurement results associated with a plurality of QC measurements performed by a plurality of medical analyzer devices on a plurality of predefined QC samples. Each QC sample is associated with a predefined target range and each medical analyzer device is associated with a peer group of medical analyzer devices. Further, the control circuitry is configured to, for each medical analyzer device, evaluate the obtained measurement results against the measurement results of the associated peer group in order to identify a deviation of at least one QC measurement parameter.

Then, if a deviation above a predefined threshold is identified, the control circuitry is further configured to obtain an action log of the medical analyzer device - where the action log comprises data indicative of any previously transmitted maintenance actions associated with the medical analyzer device - and to obtain at least one suggested maintenance action for the medical analyzer device associated with the identified deviation based on a type of QC measurement parameter that is associated with the identified deviation. Moreover, the control circuitry is configured to select a maintenance action for the medical analyzer device associated with the identified deviation from the at least one suggested maintenance action based on the obtained action log, and to transmit, to a device for managing the medical analyzer device associated with the identified deviation, data indicative of an instruction to execute the selected maintenance action for the medical analyzer device. With this aspect, similar advantages and preferred features are present as in the previously discussed aspects, and vice versa.

In some embodiments, the device for managing the medical analyzer device is configured to monitor and control one or more medical analyzer devices connected to the device.

In some embodiments, the device for managing the medical analyzer device is associated with a geographical location comprising one or more medical analyzer devices.

Further, in some embodiments, the control circuitry is configured to identify a parameter group of the type of QC measurement parameter that is associated with the identified deviation, and to obtain at least one suggested maintenance action based on the identified parameter group.

Further, in some embodiments, the at least one suggested maintenance action comprises a plurality of suggested maintenance actions arranged in a sequential order, and the selected maintenance action is a first maintenance action in accordance with the sequential order. Accordingly, in some embodiments, the control circuitry is configured to, if the deviation above the predefined threshold is identified, check the obtained action log in order to identify an entry in the action log corresponding to a transmission of the first maintenance action. Then, if the entry in the action log corresponding to the transmission of the first maintenance action is identified and if the deviation above the predefined threshold is identified at a point in time after the entry in the action log corresponding to the transmission of the first maintenance action, the control circuitry is further configured to transmit, to the device for managing the medical analyzer device associated with the identified deviation, data indicative of an instruction to execute a second maintenance action of the plurality of suggested maintenance actions for the medical analyzer device. The second action is a subsequent maintenance action relative to the first maintenance action in accordance with the sequential order.

Still further, in some embodiments, the control circuitry is configured to obtain a maintenance log of the medical analyzer device. The maintenance log comprises data indicative of historical maintenance actions performed on the medical analyzer device prior to a point in time defined by a timing of the obtained measurement results of the medical analyzer device. Then, after transmitting data indicative of the selected maintenance action for the medical analyzer device, the control circuitry is further configured to check the obtained maintenance log in order to identify an entry in the maintenance log corresponding to an execution of the selected maintenance action. Accordingly, if the entry in the maintenance log corresponding to the execution of the selected maintenance action is not identified within a predefined time period, the control circuitry is configured to transmit, to the device for managing the medical analyzer device associated with the identified deviation, data indicative of a reminder of the selected maintenance action for the medical analyzer device.

Yet further, in some embodiments, the measurement results of the peer group comprises information about an average measurement value for each QC measurement associated with the peer group. Accordingly, in some embodiments, the control circuitry is configured to update the average measurement value for each QC measurement of the peer group based on at least a portion of the obtained plurality of measurements.

In some embodiments, the predefined threshold comprises a first predefined threshold, and the control circuitry is configured to evaluate the obtained measurement results by comparing the obtained measurement results with the average measurement value for each QC measurement of the associated peer group. Thus, the control circuitry may be configured to determine that a deviation is identified if the comparison indicates a difference above the first predefined threshold.

In some embodiments, the obtained measurement results comprise information about an evolution over time of the measurement results for each of the plurality of medical analyzer devices and the predefined threshold comprises a second predefined threshold. Accordingly, in some embodiments, the control circuitry is configured to evaluate the obtained measurement results by comparing the measurement results for each QC measurement parameter for a most recent time period with an evolution over time of the average measurement value for each corresponding QC measurement of the associated peer group during the most recent time period. Thus, the control circuitry may be configured to determine that a deviation is identified if the comparison indicates a difference above the second predefined threshold value during the most recent time period

Further, in some embodiments, the obtained measurement results comprises information about an evolution over time of the measurement results for the plurality of medical analyzer devices. Accordingly, in some embodiments, the control circuitry is configured to evaluate the obtained measurement results by comparing the measurement results for each QC measurement parameter for a most recent time period with an evolution over time of the average measurement value for each corresponding QC measurement during the most recent time period. Thus, the control circuitry may be configured to determine that a deviation is identified if the comparison indicates an increasing difference over time during the most recent time period.

A fourth aspect is a remote server comprising the apparatus of the third aspect according to any one of the embodiments disclosed herein. With this aspect, similar advantages and preferred features are present as in the previously discussed aspects, and vice versa.

A fifth aspect is a cloud environment comprising one or more remote servers of the fourth aspect according to any one of the embodiments disclosed herein. With this aspect, similar advantages and preferred features are present as in the previously discussed aspects, and vice versa.

An advantage of some embodiments is that maintenance of medical analyzer devices is facilitated. In more detail, by utilizing a centralized solution for evaluating the QC measurements as disclosed herein, the maintenance needs may be identified in a more accurate and timely manner, thereby providing a time efficient maintenance solution.

An advantage of some embodiments is that the risk of erroneously replacing functional components is reduced, thereby reducing the operational costs associated with the medical analyzer devices.

An advantage of some embodiments is that down-time of the medical analyzer devices is reduced as maintenance of medical analyzer devices is facilitated.

An advantage of some embodiments is that the need for maintenance by a field service engineer is reduced.

These and other features and advantages of the embodiments described herein will in the following be further clarified with reference to the embodiments described hereinafter.

### BRIEF DESCRIPTION OF THE DRAWINGS

Further objects, features and advantages of some embodiments will appear from the following detailed description, reference being made to the accompanying drawings, in which:
Fig. 1 is schematic flowchart representation of a computer-implemented method for facilitating maintenance of a medical analyzer device in accordance with some embodiments.
Fig. 2 is a schematic flow chart representation of a computer-implemented method for facilitating maintenance of a medical analyzer device in accordance with some embodiments.
Fig. 3 is a schematic flow chart representation of some evaluation processes for collected measurement results against peer group measurements in accordance with some embodiments.
Fig. 4 is a schematic block diagram representation of a system comprising an apparatus for facilitating maintenance of a medical analyzer device in accordance with some embodiments.

### DETAILED DESCRIPTION

Those skilled in the art will appreciate that the steps, services and functions explained herein may be implemented using individual hardware circuitry, using software functioning in conjunction with a programmed microprocessor or general purpose computer, using one or more Application Specific Integrated Circuits (ASICs) and/or using one or more Digital Signal Processors (DSPs). It will also be appreciated that when embodiments are described in terms of a method, it may also be embodied in one or more processors and one or more memories coupled to the one or more processors, wherein the one or more memories store one or more programs that perform the steps, services and functions disclosed herein when executed by the one or more processors.

Embodiments of the present disclosure will be described and exemplified more fully hereinafter with reference to the accompanying drawings. The solutions disclosed herein can, however, be realized in many different forms and should not be construed as being limited to the embodiments set forth herein.

In the following, embodiments will be described for facilitating maintenance of one or more medical analyzer devices in the context of utilizing peer group measurements to identify potential issues with associated medical analyzer devices or potential negative trends with associated medical devices so that appropriate maintenance actions may be taken.

Generally, when a medical analyzer device is referred to herein, it may be understood as a device configured to conduct chemical, optical, physical or similar analysis on specimen e.g. on individual samples or sample streams. Such medical analyzer devices include analyzers for performing various forms of clinical tests and/or analysis, such as the measurement of physiological parameters of a patient.

Generally, when a "device for managing one or more medical analyzer devices" is referred to herein, it may be understood as a device associated with the hospital or facility comprising the one or more medical analyzer devices. However, the one or more medical analyzer devices may be distributed over a number of geographical locations, wherefore the "device for managing one or more medical analyzer devices" may also be associated with a plurality of hospitals or facilities comprising the one more medical analyzer devices. The "device for managing one or more medical analyzer devices" is configured to keep an overview or monitor a status and operation of the one or more medical analyzer devices. Moreover, the "device for managing one or more medical analyzer devices" is configured to transmit data (e.g. instructions) to the medical analyzer devices associated therewith.

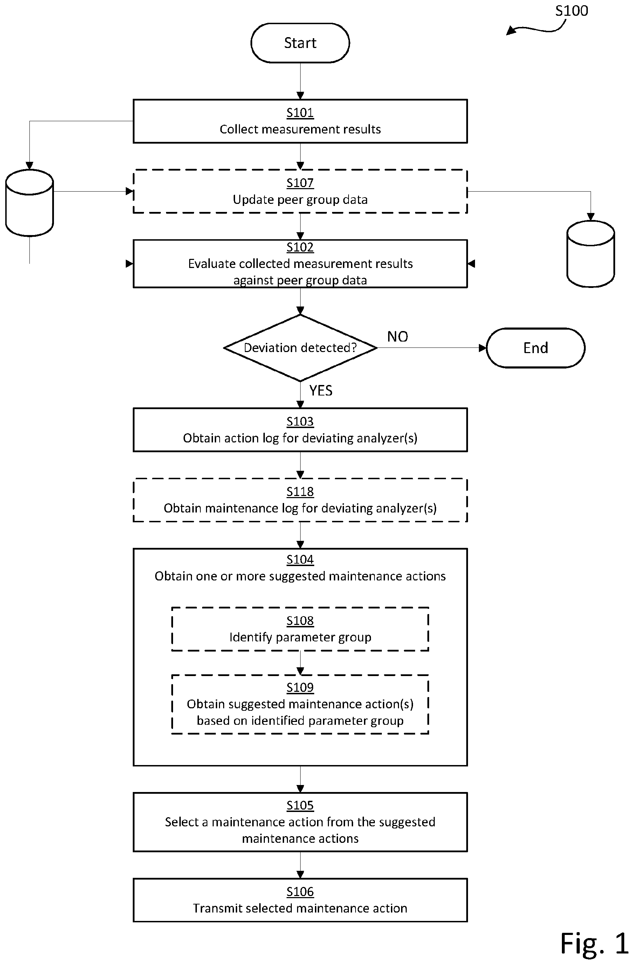

Fig. 1 is a schematic flowchart representation of a computer-implemented method S100 for facilitating maintenance of a medical analyzer device in accordance with some embodiments. A purpose of such a method may be to determine suitable actions and transmit indicators for predictive maintenance of the medical analyzer device.

The method S100 comprises obtaining S101 a plurality of measurements results associated with a plurality of quality control (QC) measurements performed by a plurality of medical analyzer devices on a plurality of predefined QC samples (may also be referred to as control substance, QC specimen or QC samples). In more detail, a QC sample may understood as a substance or sample having a measurable property that is carefully controlled such that when a medical analyzer devices performs a measurement on the QC sample to verify its operational accuracy, there is an associated target range (e.g. target value ± tolerance value) that the measurement result within which the result should fall in order to fulfil the associated quality requirements. The target ranges may for example be set by the manufacturer or distributor of the QC samples. The term obtaining is herein to be interpreted broadly and encompasses receiving, retrieving, collecting, acquiring, determining, deriving, and so forth.

Furthermore, each medical analyzer devices is associated with a peer group of medical analyzer devices. In general, a peer group of medical analyzer devices as referred to herein, may be understood as a group of medical analyzer devices that share some specific common traits. For example, a peer group of medical analyzer devices may be defined as medical analyzer devices of the same type (e.g. same model) and/or as medical analyzer devices having the same QC product type (i.e. using QC samples originating from the same source). Furthermore, the peer group may further comprise geographical constraints-e.g. even if the medical analyzer devices are of the same type they need not necessarily belong to the same peer group if they are associated with (e.g. residing in) different countries.

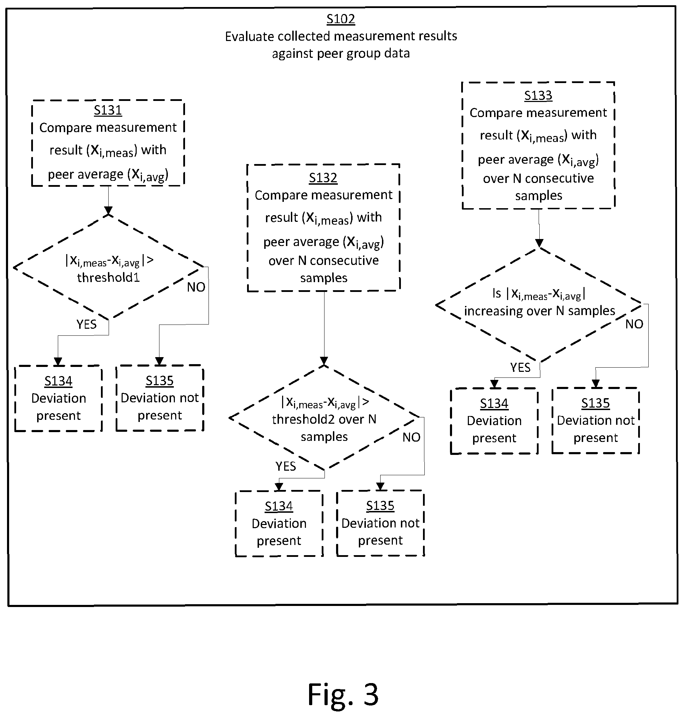

Further, the method S100 comprises, for each medical analyzer device, evaluating S102 the obtained measurement results (reference to S101) against measurement results of the associated peer group in order to identify a deviation of at least one QC measurement parameter. Some examples of an evaluation process as in S102 as well as some examples of what the term "deviating" may encompass are provided in reference to Fig. 3 below.

In general, the "measurement results of the associated peer group" may be understood as previously obtained measurement results from the associated peer group, i.e. one or more previously obtained measurement results. In some embodiments, the "measurement results of the associated peer group" comprise an average measurement result for each QC measurement of the associated peer group. Moreover, in some embodiments, the "measurement results of the associated peer group" comprises a moving average measurement result for each QC measurement of the associated peer group.

Moving on, it is determined if a deviation is detected or not, e.g., if a deviation above a predefined threshold is identified. If a deviation is detected, the method S100 further comprises obtaining S103 an action log of the (deviating) medical analyzer device(s). The action log comprises data indicative of any previously transmitted maintenance actions associated with the medical analyzer device.

Further, at least one suggested maintenance action for the medical analyzer device(s) associated with the identified deviation(s) is obtained in S104. The obtained suggested maintenance action in S104 is based on a type of QC measurement parameter that is associated with the identified deviation. For example, different suggested maintenance actions may be obtained depending on if the deviating measurement parameter is a potassium concentration (e.g. concentration of potassium ions in plasma, cK⁺) as compared to if the deviating parameter is concentration of hydrogen (e.g. concentration of hydrogen ions in blood, cH⁺).

Other QC measurement parameters include, but are not limited to - pH (measure of the acidity of alkalinity of a sample) - *p*CO₂ (partial pressure (or tension) of carbon dioxide in blood) - *p*O₂ (partial pressure (or tension) of oxygen in blood) - ctHb (concentration of total hemogloblin in blood) - _{S}O₂ (oxygen saturation) - FO₂Hb (fraction of oxyhemoglobin in total hemoglobin in blood) - FCOHb (fraction of carboxyhemoglobin in total hemoglobin in blood) - FMetHb (fraction of methemoglobin in total hemoglobin in blood) - cNa⁺ (concentration of sodium ions in plasma) - cGlu (concentration of D-glucose in plasma). This list should not be construed as exhaustive but may include further measurement parameters are readily understood by the skilled person in the art.

Moreover, each QC measurement parameter may be associated with a parameter group out of a set of predefined parameter groups. The set of predefined parameter groups may be formed based on one or more hardware and/or software components of the medical analyzer devices being associated with the performance of the QC measurement. For example, the software and/or hardware components responsible for executing the concentration measurement for sodium ions (cNa⁺) in plasma may also be responsible for executing the concentration measurement for calcium ions (cCa²⁺) in plasma. Then, these QC measurement parameters (i.e. cNa⁺ and cCa²⁺) may be associated with the same parameter group.

Accordingly, in some embodiments, the method S100 further comprises identifying S108 a parameter group of the type of QC measurement parameter that is associated with the identified deviation, and obtaining S109 the at least one suggested maintenance action based on the identified parameter group.

Further, the method S100 comprises selecting S105 a maintenance action for the medical analyzer device associated with the identified deviation from the one or more suggested maintenance actions based on the obtained action log. As mentioned, the action log is indicative of any previously transmitted maintenance actions for the medical analyzer device associated with the identified deviation. Thus, the action log may for example indicate that a first action (e.g. clean membrane X) has previously been transmitted. However, seeing that this first action did not solve the problem, then a second (subsequent) action (e.g. replace membrane X) may be selected instead.

Lastly, the method S100 comprises transmitting S106, to a device for managing the medical analyzer device associated with the identified deviation, data indicative of an instruction to execute the selected maintenance action for the medical analyzer device. As mentioned in the foregoing, the "device for managing one or more medical analyzer devices" may be understood as a device configured to keep an overview or monitor a status and operation of the one or more medical analyzer devices, and further to receive data from and transmit instructions to the one or more medical analyzer devices.

As mentioned, the measurement results of the associated peer group may comprise information about an average measurement value for each QC measurement for that peer group. Therefore, the method S100 may further comprise updating S107 the average measurement value for each QC measurement of the peer group based on at least a portion of the obtained plurality of measurements.

Moreover, the obtained S101 measurement results of each medical analyzer device may for example be in the form of an average measurement value for each QC sample. In more detail, the average measurement value for each QC sample may for example be an average value based on the last ten measurements for each QC sample. However, in some embodiments, the method S100 comprises obtaining the N latest measurement results associated with the plurality of QC measurements performed by the plurality of medical analyzer devices (N ≥ 2), and averaging the obtained N latest measurements in order to determine the average measurement value for each QC sample for each medical analyzer device. Moreover, the method S100 may further comprise filtering the obtained N latest measurements before determining the average measurement value in order to remove any obvious erroneous measurements from the formed average value. An advantage with the filtering and averaging of the QC measurements results of each medical analyzer device is that the risk of issuing unnecessary maintenance actions is reduced. Another advantage with the filtering and averaging of the QC measurements of each medical analyzer device is that the risk of including statistical outliers (i.e. obvious erroneous entries) in the peer group data is reduced.

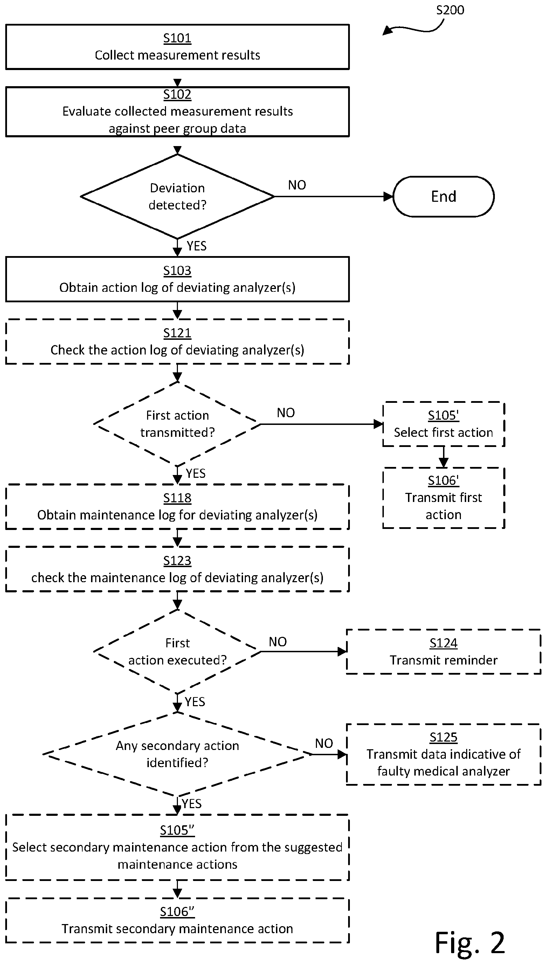

Moreover, in some embodiments, each medical analyzer device is associated with a maintenance log. Thus, the method S100 may further comprise obtaining S118 a maintenance log of the medical analyzer device. The maintenance log comprises data indicative of historical maintenance actions performed on the (deviating) medical analyzer device prior to a point in time defined by a timing of the obtained measurement results of the (deviating) medical analyzer device. The maintenance log is suitably used for monitoring the performed maintenance actions on the medical analyzer devices in order to issue reminders in case a transmitted maintenance action has not been performed. Some example embodiments related to the use cases with the action log and the maintenance log are exemplified in Fig. 2

Thus, turning to Fig. 2, there is illustrated a schematic flow chart representation of a computer-implemented method S200 for facilitating maintenance of a medical analyzer device in accordance with some embodiments. As indicated in Fig. 2, some of the steps correspond to the analogous steps as in Fig. 1, and will for the sake of brevity and conciseness not be elaborated upon in any greater detail.

Accordingly, the method S200 comprises obtaining or collecting S101 QC measurement results from a plurality of medical analyzer devices and evaluating S102 the collected S101 measurement results against the measurement results of the associated peer groups. The evaluation in S102 is performed in order to detect any potentially malfunctioning or erroneously operating medical analyzer devices based on a comparison between the QC measurement results of each medical analyzer device in view of the measurement results of the associate peer groups.

Then, if at least one QC measurement parameter is deviating, above a threshold, from the measurement results of the peer group, the action log of those deviating medical analyzers is obtained S103. As mentioned in the foregoing, in some embodiments, there is a plurality of suggested maintenance actions that may be selected for an identified/detected deviation. These suggested maintenance actions may be arranged in a sequential order - i.e. a first maintenance action, a second maintenance action, and so forth.

Thus, in some embodiments, the method S200 may further comprise checking S121 the obtained S103 action log of the deviating medical analyzer device in order to identify an entry in the obtained S103 action log corresponding to a transmission of the first maintenance action. If there is no entry corresponding to the first suggested maintenance, the method S200 further comprises selecting S105' the first maintenance action and transmitting S106', to the device for managing the medical analyzer device associated with the identified deviation, data indicative of an instruction to execute the first maintenance action.

However, in some cases the check S121 of the action log may indicate an entry in the action log corresponding to the transmission of the first maintenance action. Assuming that the deviation above the predefined threshold is identified at a point in time after the entry in the action log corresponding to the transmission of the first maintenance action, the method S200 may further comprise selecting S105" the second/next maintenance action from the plurality of suggested maintenance actions. Then, data indicative of an instruction to execute a second maintenance action of the plurality of suggested maintenance actions for the medical analyzer device is transmitted S106" to the device for managing the medical analyzer device associated with the identified deviation. The second action may therefore be understood as a subsequent maintenance action relative to the first maintenance action in accordance with the sequential order.

In other words, the action log may be understood as a log comprising a maintenance process for the deviating medical analyzer device and furthermore indicative of which step the associated medical analyzer device currently is at in that maintenance process. Thereby one is able to keep track of the maintenance process for a vast number of medical analyzer devices, reducing the risk of issuing instructions of inappropriate maintenance actions.

Moreover, in some embodiments, each of the medical analyzer devices are associated with a maintenance log comprising data indicative of historical maintenance actions performed on the medical analyzer device prior to a point in time defined by a timing of the obtained measurement results of the medical analyzer device.

Accordingly, in some embodiments, the method S200 comprises obtaining S118 the maintenance log of the deviating medical analyzer, and checking S123 the obtained maintenance log in order to identify an entry in the maintenance log corresponding to an execution of the selected and transmitted maintenance action. If there is no entry in the maintenance log corresponding to the execution of the transmitted and selected maintenance action, the method S200 may further comprise transmitting S124 data indicative of a reminder of the selected maintenance action for the medical analyzer device to the device for managing the medical analyzer device associated with the identified deviation.

However, if there is an entry in the maintenance log corresponding to the execution of the transmitted and selected maintenance action, and if there is no second/next maintenance action in the action log, the method S200 may further comprise transmitting S125 data indicative of a faulty medical analyzer device to the device for managing the medical analyzer device associated with the identified deviation. This may for example be interpreted as an indication of the medical analyzer device to be taken out of function and that dedicated service personnel, e.g. field service engineers. should be contacted to repair the faulty medical analyzer device.

Some examples of what the step of evaluating S102 the obtained measurement results against measurement results of the associated peer group may comprise in accordance with some embodiments will now be provided in reference to Fig. 3. In more detail, Fig. 3 shows some flow chart representations of the evaluation S102 process in accordance with some embodiments.

As mentioned in the foregoing, the measurement results of the peer groups may be in the form of average measurement values (e.g. 6 month rolling average values) for each QC measurement. Accordingly, in some embodiments, the step of evaluating S102 the obtained measurement results comprises comparing S131 the obtained measurement results (X_{i, meas}) with the average measurement value for each QC measurement parameter (Xᵢ, _{avg}) of the associated peer group. Here "I" denotes the type of QC sample. In other words, the obtained measurement results for e.g. the concentration of calcium ions (cCa²⁺) in plasma as measured by a medical analyzer device is compared with the peer average of the concentration of calcium ions (cCa²⁺) in plasma. Then, the method further comprises determining S134 that a deviation is identified/present, for a specific QC measurement parameter, if the comparison S131 indicates a difference above a first predefined threshold (|x_{i,meas}-X_{i,avg}| > threshold1) for that specific QC measurement parameter. If the comparison S131 would indicate a difference below the first predefined threshold (|X_{i,meas}-X_{i,avg}| ≤ threshold1), it is concluded S135 that no deviation is present. Further, in some embodiments, the obtained measurement results comprise information about an evolution over time of the measurement results for each of the plurality of medical analyzer devices and wherein the predefined threshold comprises a second predefined threshold. Accordingly, in some embodiments, the step of evaluating the obtained measurement results comparing S132 the obtained measurement results (X_{i, meas}) for each QC measurement parameter for a most recent time period with an evolution over time of the average measurement value for each corresponding QC measurement (Xᵢ, _{avg}) of the associated peer group during the most recent time period. In other words, the measurement results (X_{i, meas}) for each QC measurement parameter is compared S132 with the associated peer average (Xᵢ, _{avg}) over N consecutive samples. Then, the method further comprises determining S134 that a deviation is identified/present, for a specific QC measurement parameter, if the comparison S131 indicates a difference above a second predefined threshold (|X_{i,meas}-_{Xi,avg}| > threshold2) for that specific QC measurement parameter during the most recent time period. If the comparison S131 would indicate a difference below the second predefined threshold (|x_{i,meas}-X_{i,avg}| ≤ threshold2) during the most recent time period, it is concluded S135 that no deviation is present.

Still further, in some embodiments, the obtained measurement results comprises information about an evolution over time of the measurement results for the plurality of medical analyzer devices. Accordingly, in some embodiments, the step of evaluating the obtained measurement results comparing S133 the obtained measurement results (Xᵢ, ₘₑₐₛ) for each QC measurement parameter for a most recent time period with an evolution over time of the average measurement value for each corresponding QC measurement (Xᵢ, _{avg}) of the associated peer group during the most recent time period. In other words, the measurement results (X_{i, meas}) for each QC measurement parameter is compared S133 with the associated peer average (Xᵢ, _{avg}) over N consecutive samples. Then, the method further comprises determining S134 that a deviation is identified/present, for a specific QC measurement parameter, if the comparison S131 indicates an increasing difference over time for that specific QC measurement parameter during the most recent time period. If the comparison S131 would not indicate an increasing difference over time for that specific QC measurement parameter during the most recent time period, it is concluded S135 that no deviation is present.

Executable instructions for performing these functions and steps are, optionally, included in a non-transitory computer-readable storage medium or other computer program product configured for execution by one or more processors.

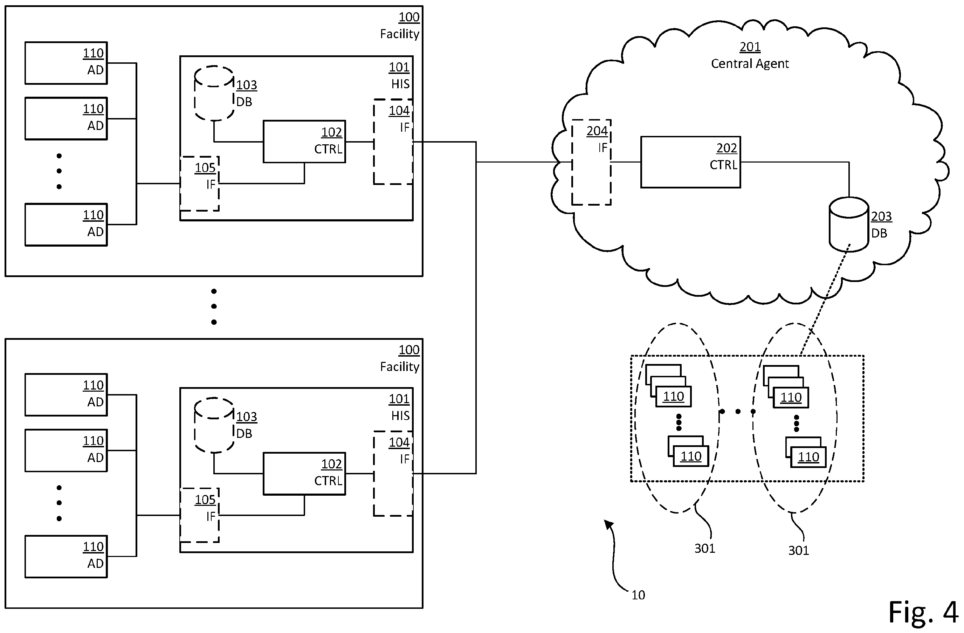

Fig. 4 is a schematic block diagram representation of a system 10 comprising an apparatus 201 for facilitating maintenance of a medical analyzer device 110 in accordance with some embodiments. The apparatus 201 comprises control circuitry CTRL 202 configured to obtain a plurality of measurement results associated with a plurality of quality control (QC) measurements performed by a plurality of medical analyzer devices 110 on a plurality of predefined QC samples. Each QC sample is associated with a predefined target range and each medical analyzer device 110 is associated with a peer group 301 of medical analyzer devices.

Further, for each medical analyzer device 110, the control circuitry 202 is configured to evaluate the obtained measurement results against the measurement results of the associated peer group in order to identify a deviation of at least one QC measurement parameter. Then, if a deviation above a predefined threshold is identified, the control circuitry 202 is further configured to obtain an action log(s) of the deviating medical analyzer device(s) 110. The action log(s) comprise(s) data indicative of any previously transmitted maintenance actions associated with the medical analyzer device(s) 110.

The control circuitry 202 is further configured to obtain at least one suggested maintenance action for the deviating medical analyzer device 110, i.e. for the medical analyzer device associated with the identified deviation. The obtained one or more suggested maintenance actions are based on a type of QC measurement parameter that is associated with the identified deviation.

Further, the control circuitry 202 is configured to select a maintenance action for the medical analyzer device associated with the identified deviation from the one or more suggested maintenance actions based on the obtained action log. Then, the control circuitry 202 is configured to transmit to a device 101 for managing the medical analyzer device 110 associated with the identified deviation, data indicative of an instruction to execute the selected maintenance action for the medical analyzer device.

In some embodiments, the device 101 for managing the medical analyzer device has control circuitry 102 configured to monitor and control one or more medical analyzer devices connected to the device 101. Thus, the device 101 for managing the medical analyzer devices may be part of a Hospital Information System (HIS) and/or a Laboratory Information System (LIS), responsible for monitoring and controlling a plurality of medical analyzer devices associated thereto. Thus, in some embodiments, the device 101 for managing the medical analyzer device is associated with a geographical location comprising one or more medical analyzer devices. Nevertheless, in some embodiments, the device may be in the form of (e.g., implemented as) a cloud service (provided by a cloud environment, e.g., a cloud computing system, comprising one or more remote servers, e.g., distributed cloud computing resources) communicatively connected to one or more medical analyzer devices.

In some embodiments, the device 101 for managing a plurality of medical analyzer devices 110 has control circuitry 102 configured to obtain a plurality of measurement results associated with a plurality of QC measurements performed by one or more of the plurality of medical analyzer devices 110 on a set of predefined QC samples. The plurality of medical analyzer and the device 101 may for example be associated with the same facility (e.g. hospital or laboratory).

Further, the control circuitry 102 is configured to transmit, to a central agent 201 (e.g. the apparatus described in the foregoing), data indicative of the obtained plurality of measurement results. The control circuitry 102 may be further configured to transmit data indicative of the analyzer type and the QC product types of each medical analyzer device of the plurality of medical analyzer devices.

In some embodiments, the control circuitry 102 is further configured to receive, from the central agent 201, data indicative of an instruction to execute a selected maintenance action for the one or more medical analyzer devices 110 associated thereto.

However, in some embodiments, the control circuitry 202 may be configured to transmit the data indicative of an instruction to execute a selected maintenance action directly to each medical analyzer device 110. Accordingly, each medical analyzer device 110 may comprise control circuitry configured to (individually or independently) transmit data indicative of the obtained plurality of measurement results to the central agent 201. In other words, in some embodiments, the device 101 for managing the medical analyzer device may be an internal control unit or control circuitry of the medical analyzer device 110.

Other embodiments than the above described are possible and within the scope of the claims. Different method steps than those described above, performing the method by hardware or software, may be provided within the scope of the embodiments disclosed herein. Thus, according to an exemplary embodiment, there is provided a non-transitory computer-readable storage medium storing one or more programs configured to be executed by one or more processors of a processing system, the one or more programs comprising instructions for performing the method according to any one of the above-discussed embodiments. Alternatively, according to another exemplary embodiment a cloud computing system can be configured to perform any of the methods presented herein. The cloud computing system may comprise distributed cloud computing resources that jointly perform the methods presented herein under control of one or more computer program products.

Generally speaking, a computer-accessible medium may include any tangible or non-transitory storage media or memory media such as electronic, magnetic, or optical media-e.g., disk or CD/DVD-ROM coupled to computer system via bus. The terms "tangible" and "non-transitory," as used herein, are intended to describe a computer-readable storage medium (or "memory") excluding propagating electromagnetic signals, but are not intended to otherwise limit the type of physical computer-readable storage device that is encompassed by the phrase computer-readable medium or memory. For instance, the terms "non-transitory computer-readable medium" or "tangible memory" are intended to encompass types of storage devices that do not necessarily store information permanently, including for example, random access memory (RAM). Program instructions and data stored on a tangible computer-accessible storage medium in non-transitory form may further be transmitted by transmission media or signals such as electrical, electromagnetic, or digital signals, which may be conveyed via a communication medium such as a network and/or a wireless link.

The processor(s) 102, 202 (associated with the apparatuses and devices 101, 201) may be or include any number of hardware components for conducting data or signal processing or for executing computer code stored in memory 103, 203. The apparatus and device 101, 201 have an associated memory 103, 203, and the memory 103, 203 may be one or more devices for storing data and/or computer code for completing or facilitating the various methods described in the present description. The memory may include volatile memory or non-volatile memory. The memory 103, 203 may include database components, object code components, script components, or any other type of information structure for supporting the various activities of the present description. According to an exemplary embodiment, any distributed or local memory device may be utilized with the apparatuses and methods of this description. According to an exemplary embodiment the memory 103, 203 is communicably connected to the processor 102, 202 (e.g., via a circuit or any other wired, wireless, or network connection) and includes computer code for executing one or more processes described herein.

The communication interfaces 104, 105 204 may provide the possibility to send output to a remote location (e.g. remote operator or control centre) by means of a suitable communication arrangement. The communication interfaces 104, 105, 204 may be arranged to communicate with other functional components and may thus be seen as control interface also; however, a separate control interface (not shown) may be provided. Local communication within the facility 100 may also be of a wireless type with protocols such as WiFi, LoRa, Zigbee, Bluetooth, or similar mid/short range technologies.

Accordingly, it should be understood that parts of the described solution may be implemented either in a remote server, or in plurality of remote servers, for instance in a plurality of servers in communication with each other, a so called cloud solution. The different features and steps of the embodiments may be combined in other combinations than those described.

Generally, all terms used herein are to be interpreted according to their ordinary meaning in the relevant technical field, unless a different meaning is clearly given and/or is implied from the context in which it is used.

It should be noted that the word "comprising" does not exclude the presence of other elements or steps than those listed and the words "a" or "an" preceding an element do not exclude the presence of a plurality of such elements. It should further be noted that any reference signs do not limit the scope of the claims, that the embodiments may be at least in part implemented by means of both hardware and software, and that several "control circuits", "means" or "units" may be represented by the same item of hardware.

Although the figures may show a specific order of method steps, the order of the steps may differ from what is depicted. In addition, two or more steps may be performed concurrently or with partial concurrence. Such variation will depend on the software and hardware systems chosen and on designer choice. All such variations are within the scope of the disclosed embodiments. Likewise, software implementations could be accomplished with standard programming techniques with rule-based logic and other logic to accomplish the various connection steps, processing steps, comparison steps and decision steps. The above mentioned and described embodiments are only given as examples and should not be limiting to the present disclosure. Hence, it should be understood that the details of the described embodiments are merely examples brought forward for illustrative purposes, and that all variations that fall within the scope of the claims are intended to be embraced therein.

## Claims

1. A computer-implemented method (S100, S200) for facilitating maintenance of a medical analyzer device, the method comprising:
obtaining (S101) a plurality of measurement results associated with a plurality of quality control, QC, measurements performed by a plurality of medical analyzer devices on a plurality of predefined QC samples, wherein each QC sample is associated with a predefined target range and wherein each medical analyzer device is associated with a peer group of medical analyzer devices;
for each medical analyzer device:
evaluating (S102) the obtained measurement results against measurement results of the associated peer group in order to identify a deviation of at least one QC measurement parameter;
if a deviation above a predefined threshold is identified:
obtaining (S103) an action log of the medical analyzer device, the action log comprising data indicative of any previously transmitted maintenance actions associated with the medical analyzer device;
obtaining (S104) at least one suggested maintenance action for the medical analyzer device associated with the identified deviation based on a type of QC measurement parameter that is associated with the identified deviation;
selecting (S105, S105', S105") a maintenance action for the medical analyzer device associated with the identified deviation from the at least one suggested maintenance action based on the obtained action log;
transmitting (S106, S106', S106"), to a device for managing the medical analyzer device associated with the identified deviation, data indicative of an instruction to execute the selected maintenance action for the medical analyzer device.

2. The method (S100, S200) according to claim 1, wherein the step of obtaining (104) at least one maintenance action for the medical analyzer device associated with the identified deviation comprises:
identifying (S108) a parameter group of the type of QC measurement parameter that is associated with the identified deviation;
obtaining (S109) at least one suggested maintenance action based on the identified parameter group.

3. The method (S100, S200) according to claim 2, wherein each QC measurement parameter is associated with a parameter group out of a set of predefined parameter groups, and wherein the set of predefined parameter groups are formed based on one or more hardware and/or software components of the medical analyzer devices being associated with the performance of the QC measurement.

4. The method (S100, S200) according to any one of claims 1- 3, wherein the at least one suggested maintenance action comprises a plurality of suggested maintenance actions arranged in a sequential order, and wherein the selected maintenance action is a first maintenance action in accordance with the sequential order, the method (S100) further comprising:
if a deviation above the predefined threshold is identified:
checking (S121) the obtained action log in order to identify an entry in the action log corresponding to a transmission of the first maintenance action;
if the entry in the action log corresponding to the transmission of the first maintenance action is identified and if the deviation above the predefined threshold is identified at a point in time after the entry in the action log corresponding to the transmission of the first maintenance action:
transmitting (S106"), to the device for managing the medical analyzer device associated with the identified deviation, data indicative of an instruction to execute a second maintenance action of the plurality of suggested maintenance actions for the medical analyzer device, the second action being a subsequent maintenance action relative to the first maintenance action in accordance with the sequential order.

5. The method (S100, S200) according to any one of claims 1-4, further comprising:
obtaining (S118) a maintenance log of the medical analyzer device, the maintenance log comprising data indicative of historical maintenance actions performed on the medical analyzer device prior to a point in time defined by a timing of the obtained measurement results of the medical analyzer device;
after transmitting (S106, 106', 106") data indicative of the selected maintenance action for the medical analyzer device:
checking (S123) the obtained maintenance log in order to identify an entry in the maintenance log corresponding to an execution of the selected maintenance action;
if the entry in the maintenance log corresponding to the execution of the selected maintenance action is not identified within a predefined time period;
transmitting (S124), to the device for managing the medical analyzer device associated with the identified deviation, data indicative of a reminder of the selected maintenance action for the medical analyzer device.

6. The method (S100, S200) according to any one of claims 1-5, wherein the measurement results of the peer group comprises information about an average measurement value for each QC measurement associated with the peer group, the method (S100) further comprising:
updating (S107) the average measurement value for each QC measurement of the peer group based on at least a portion of the obtained plurality of measurements.

7. The method (S100, S200) according to claim 6, wherein the predefined threshold comprises a first predefined threshold, and wherein the step of evaluating (S102) the obtained measurement results comprises:
comparing (S131) the obtained measurement results with the average measurement value for each QC measurement of the associated peer group;
determining (S134) that a deviation is identified if the comparison indicates a difference above the first predefined threshold.

8. The method (S100, S200) according to any one of claims 6-7, wherein the obtained measurement results comprise information about an evolution over time of the measurement results for each of the plurality of medical analyzer devices and wherein the predefined threshold comprises a second predefined threshold, and wherein the step of evaluating (S102) the obtained measurement results comprises:
comparing (S132) the measurement results for each QC measurement parameter for a most recent time period with an evolution over time of the average measurement value for each corresponding QC measurement of the associated peer group during the most recent time period;
determining (S134) that a deviation is identified if the comparison indicates a difference above the second predefined threshold value during the most recent time period.

9. The method (S100, S200) according to any one of claims 6-8, wherein the obtained measurement results comprises information about an evolution over time of the measurement results for the plurality of medical analyzer devices, and wherein the step of evaluating (S102) the obtained measurement results comprises:
comparing (S133) the measurement results for each QC measurement parameter for a most recent time period with an evolution over time of the average measurement value for each corresponding QC measurement during the most recent time period;
determining (S134) that a deviation is identified if the comparison indicates an increasing difference over time during the most recent time period.

10. A computer-readable storage medium storing one or more programs configured to be executed by one or more processors of a processing system, the one or more programs comprising instructions for performing the method (S100, S200) according to any one of claims 1-9.

11. An apparatus (201) for facilitating maintenance of a medical analyzer device, the apparatus comprising control circuitry (202) configured to:
obtain a plurality of measurement results associated with a plurality of quality control, QC, measurements performed by a plurality of medical analyzer devices (110) on a plurality of predefined QC samples, wherein each QC sample is associated with a predefined target range and wherein each medical analyzer device (110) is associated with a peer group of medical analyzer devices;
for each medical analyzer device (110):
evaluate the obtained measurement results against the measurement results of the associated peer group in order to identify a deviation of at least one QC measurement parameter;
if a deviation above a predefined threshold is identified:
obtain an action log of the medical analyzer device (110), the action log comprising data indicative of any previously transmitted maintenance actions associated with the medical analyzer device;
obtain at least one suggested maintenance action for the medical analyzer device (110) associated with the identified deviation based on a type of QC measurement parameter that is associated with the identified deviation;
select a maintenance action for the medical analyzer device (110) associated with the identified deviation from the at least one suggested maintenance action based on the obtained action log;
transmit, to a device (101) for managing the medical analyzer device (110) associated with the identified deviation, data indicative of an instruction to execute the selected maintenance action for the medical analyzer device.

12. The apparatus according to claim 11, wherein the device for managing the medical analyzer device is configured to monitor and control one or more medical analyzer devices connected to the device.

13. The apparatus according to any of claims 11-12, wherein the device for managing the medical analyzer device is associated with a geographical location comprising one or more medical analyzer devices.

14. A remote server comprising the apparatus according to any of claims 11-13.

15. A cloud environment comprising one or more remote servers according to claim 14.
